# NEW EUROPEAN PATENT SPECIFICATION

(11) **EP 1 200 121 B2**
(45) Date of publication and mention of the opposition decision: **09.05.2012**
(45) Mention of the grant of the patent: 03.09.2008
(21) Application number: 00949768.6
(22) Date of filing: 07.08.2000
(51) Int. Cl.: A61K 39/106, A61K 39/102, A61K 39/02, C12N 1/20, C07K 16/12, A61P 31/04

(54) **FISH VACCINE**
FISCH-IMPFSTOFF
VACCIN POUR POISSON

(30) Priority: 07.08.1999 GB 9918591; 30.09.1999 GB 9923030
(43) Date of publication of application: 02.05.2002
(73) Proprietor: Novartis AG, 4056 Basel (CH); Novartis Pharma GmbH, 1230 Wien (AT)
(72) Inventor: BARNES, Andrew, Cartner, Aberdeen AB10 6RX (GB)
(74) Representative: Kamibayashi, Lynne
(86) International application number: PCT/GB2000/002973
(87) International publication number: WO 2001/010459

(56) References cited:
- WO-A-96/12734

## Description

The present invention relates to novel compositions for use as vaccines and medicaments for the prophylactic treatment of fish for infection by bacterial organisms, and particularly for the protection of fish from infection by bacteria such as *Photobacterium damselae* subsp. *piscicida.*

*Photobacterium damselae* subsp *piscicida,* formerly *Photobacterium damsela* subsp. *piscicida* was reclassified from, and formerly reported as *Pasteurella piscicida* and reallocated to the family vibrionaceae based on small subunit 16s ribosomal RNA homology (Gaulthier et al (1995) Int. J. Syst. Bacteriol. 45:139-144). *P. damselae* causes serious fatal disease, commonly called pseudotuberculosis, in warm water marine fish infecting major organs and tissues resulting in characteristic lesions or pseudotubercles in the musculature.

Appearance of antibiotic resistance coupled with the observation that fish cease feeding when infected, and thus do not consume antibiotic medicated feeds have been suggested as reasons for chemotherapeutic failure. Furthermore, there is an increasing body of evidence to suggest a phase of intracellular survival during host colonisation (Barnes et al. (1999) Microbiology 145: 483-494). Therefore prophylaxis through immunisation appears to be the most desirable means of controlling this disease.

Various vaccination strategies have been suggested based on formalin killed bacteria (bacterins), however, many of these have failed to yield reproducible protective efficacy (Romalde & Magarinos (1997) Fish Vaccinology, Karger, Basel, Switzerland, pp167-177). This may reflect the low antibody response of the host to the infectious agent (Arijo et al. (1997) Fish Shellfish Immunol. 8: 63-72). Furthermore, little is known of the protective antigens contained in these preparations. Preparations based on iron restricted outer membrane proteins (IROMPs) and bacterins enriched with extracellular products (ECP) have been described (Magarinos et al. (1994) Bull. Eur. Ass. Fish Pathol.14:120-122) but these have not been effective in commercial situations.

It is an established fact that bacteria have an absolute requirement for iron, and *P*. *damselae* is no exception. It has been shown that under conditions in which iron is limiting, either by not including sufficient in the medium, or by including chelating agents which reduce its avilability, a variety of high affinity, iron uptake systems are expressed (Magarinos et al. (1994) Appl. Environ. Microbiol. 60: 2990-2998). These include siderophores and iron restricted outer membrane proteins (IROMPS). In other bacterial species such as *Aeromonas salmonicida* (Hirst & Ellis (1994) Fish Shellfish Immunol. 4:29-45) and *Pasteurella haemolytica* (UK patent specification 8805253 W Donachie, UK) IROMPs have been exploited as protective antigens in successful vaccines. However these do not appear to be protective in *P*. *damselae,* (Romalde & Magari-os (1997) Fish Vaccinology, Karger, Basel, Switzerland, pp167-177). This lack of protection may reflect the strategy for infection by *P. damselae.* Under iron limitation, *P. damselae* produces increased protease activity (Bakopolous (1997) J. Fish Dis. 20: 297-305) and increased capsular polysaccharide. Capsular polysaccharide increases resistance to serum killing and prevents phagocytosis by host macrophages (Arijo et al. (1998) Fish Shellfish Immunol. 8: 63-72.). This is significant as *P. damselae* is not resistant to attack by phagocytes (Skarmeta et al. (1995) Dis. Aquat. Org. 23:51-57) as a result of its lack of adaptive response to killing by reactive oxygen species produced during the macrophage respiratory burst (Barnes et al. (1999) Microbiology 145: 483-495). However, the increased proteolytic activity is able to free iron in the form of haem through red blood cell lysis. Haem is absorbed by the capsule (Ana Do Vale, Pers. Comm.) and the iron translocated across the membrane by the IROMPs. Thus with capsular polysaccharide acting as the first level of iron uptake the IROMPs are not exposed during the disease process. As iron levels increase, capsular production decreases, IROMPs are switched off and proteolytic activity becomes undetectable (Bakopolous (1997) J. Fish Dis. 20: 297-305). This would leave *P*. *damselae* susceptible to both antibody and macrophage attack.

However, at this point the organism is able to adhere to (Yoshida et al. (1997) J. Fish Dis. 20: 77-80) and enter non-phagocytic fish cells (Magari-os et al. (1996) FEMS Microbiology Lett. 138: 29-34) thus avoiding attack by antibodies or phagocytes.

It is an aim of the present invention to provide a vaccine for the protection of fish from infection by bacteria such as *Photobacterium damselae.*

It is another aim of the invention to provide a process for the production of such a vaccine.

According to the present invention there is provided a vaccine and/or therapeutic composition comprising biological material derived from a culture of Photobacterium damselae, characterised in that the bacterial cells have been cultured in a culture medium containing excess iron over that which is required for normal growth of the bacterium.

By "containing excess iron" the culture medium should contain at least twice the amount of iron in standard tryptone soya broth (Oxoid).

Preferably the culture medium contains between 0.5 µM and 1mM iron.

More preferably the medium contains between 10µM and 500µM iron.

Most preferably the medium contains between 25µM and 500 µM iron.

Typically an outermembrane protein which may be involved in entry into host cells or invasin or adhesin is expressed at levels higher than those in normal culture medium.

By "expressed at levels higher than those in normal culture medium" the protein or invasin or adhesin is expressed at at least twice the normal expression levels. Normal expression levels are levels of expression in standard tryptone soya broth (Oxoid).

Typically an extracellular protein serologically related to invasin is expressed at higher levels than in normal culture.

Typically a 55Kda extracellular protein complex and/or a 97Kda outermembrane protein is expressed at levels higher than those in normal culture medium.

The invention also provides purified proteins as described for use as vaccines or in the preparation of vaccines.

The invention also provides antibodies to the purified proteins.

The invention also provides a method for production wherein the cells are cultured in a culture medium containing sufficient iron such that the cells are loaded up with iron to the point where linear uptake of iron from the culture medium no longer occurs.

Preferably the cells are saturated with iron.

Preferably iron is supplied to the medium in the form of a ferric salt.

Preferably the bacteria have been inactivated after culture.

Preferably the inactivation is carried out using a formaldehyde composition.

The invention further provides cells or cell membranes or extracellular products of the cultured bacteria.

The biological material is preferably provided in a physiologically acceptable carrier.

The composition of the invention preferably includes an adjuvant suitable for enhancing immunological response.

A vaccine composition is thus provided for the prophylactic and/or therapeutic treatment of fish for infection by bacteria, particularly by the organism Photobacterium damelae subsp. piscicida.

The composition comprises components produced by a culture of the organism. The culture is treated to kill the organism prior to use, preferably by treatment with formalin after components have been produced.

Two of the components, involved in invasion of host cells and produced in high quantities when the culture is grown in medium containing excess iron, induce production of antibodies on injection into fish which prevent the entry of the organism into fish cells.

These antibodies protect the fish from infection by the organism.

The invention thus also provides antibodies.

In one particular embodiment the biological material is derived from the culture of *Photobacterium damselae* strain MT1415 deposited under Accession No 41062 on 4 August 2000 at N.C.I.M.B. in Aberdeen, United Kingdom.

The present inventor has determined that a protein (invasin or adhesin) expressed in the outer membrane under iron replete conditions is involved with internalisation of *P. damselae* by Sea Bass Fibroblastic Cells. Lectins which specifically bind this protein inhibit the invasive capacity of *P. damselae,* whilst lectins which do not bind the protein do not significantly inhibit invasion of Sea Bass Larval fibroblast cell lines (SBLs). Furthermore, antibodies raised against this protein in sea bass prevent internalisation of the bacterium by fish epithelial cells (EPCs). The expression of this protein can be increased by addition of ferric iron in the form of ferric chloride, to the growth medium. For example, addition of one hundred micromolar (100 µM) ferric chloride increases expression of the protein by at least 2.6 fold. Bacterins produced by growing *P. damselae* under such iron supplementation are protective against clinical pasteurellosis in rainbow trout and juvenile sea bream.

Iron may be supplied to the culture medium in any form that results in its uptake by the bacterial cells in such a manner that increase of the invasin results. Other culture conditions may also increase expression of this protein. For example more may be expressed under anaerobiosis in the presence of sufficient iron. However, higher cell densities are achieved by growing the bacteria between 22°C and 35°C with continous shaking, or other aeration, to maintain the oxygen content.

The vaccine cells of the invention are inactivated by any standard method, but conveniently by use of formaldehyde. Further preparation of the vaccine such as addition of an adjuvant, concentration of the cells, or resuspension into an acceptable carrier may afford additional protection.

The vaccines of the invention and a method for their production will now be described by way of illustration only by reference to the following nonlimiting Examples and Figures. Further embodiments falling within the scope of the claims will occur to those skilled in the art in light of these.

### Figures

Figure 1. Shows a histogram of relative percent survival (RPS) vs vaccine treatment for rainbow trout challenged with organisms of genus *Photobacterium.*

Figure 2. Shows a histogram of RPS vs vaccine treatment for gilthead seabream (Sparus aurata) challenged with organisms of genus *Photobacterium.*

Figure 3. Shows a Western blot of *P. damselae* outer membrane proteins (OMP, lanes 2,4,6,8) and extracellular products (ECP, lanes 3,5,7,9) stained with aurodye (for protein, lanes 1-5) and sea bass vs iron-supplemented *P. damselae* (lanes 6-9), Clearly showing 97KDa protein (lane 8) and 55Kda protein complex (lanes 7, 9) .

Figure 4. Shows percentage of EPC cells with at least one intracellular *P. damselea* preincubated with 2) normal sea bass serum or 3) sea bass vs *P. damselae* invasin antiserum with 1) control.

Figure 5. Shows invasion of EPC/SBL cells by *P. damselae* incubated with various lectins, relative to controls not previously incubated with lectins.

Figure 6. Shows Western blot of *P. damselae* OMPs produced under iron supplemented conditions, probed with Lectins and sea bass vs *P. damselae* antiserum and rabbit anti 55KDa complex.Lanes: 1) rabbit vs 55KDa complex antibody; 2) bass vs MT1415 antibody 3) biotinylated dolichos biflorus agglutinin; 4) biotinylated Concanavalin A lectin; 5) Sigma biotinylated molecular weight markers.

Figure 7 shows Western blot, stained with Sea Bass vs Photobacterium damselae antibody, of Outer membrane proteins from vaccine preparations showing absence of 55KDa complex in iron depleted culture (Vaccine B), compared to iron supplemented culture (Vaccine A).

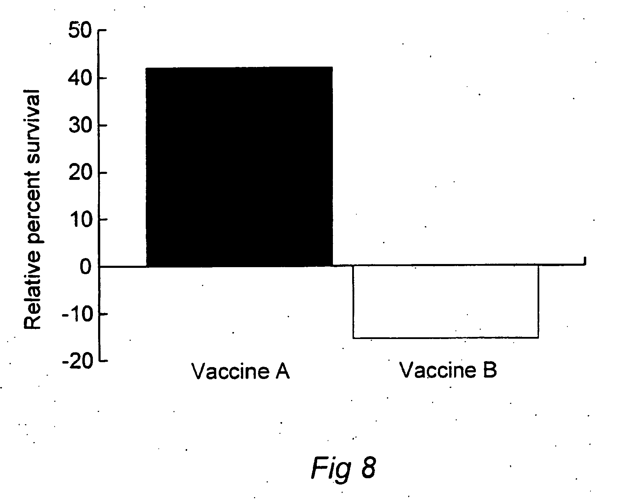

Figure 8 shows relative percent survival (RPS) of Sea bass Dicentrarclus Labrax challenged with Photobacterium damselae subsp. piscicida following vaccination with either A)Vaccine expressing 55KDa complex and 97KDa OMP or B) Vaccine NOT expressing 55KDa complex or 97KDa OMP (see also Figure 7).

### Example 1: Production of an invasin-expressing P. damselae vaccine

Tryptone soya broth (Oxoid) was made up in distilled water at 30g/l and sodium chloride was added at 20g/l in conical flasks such that the volume of the flask was five times the volume of the medium to allow for sufficent aeration. After sterilisation by autoclaving at 121 °C for 15 minutes, ferric chloride was added to a final concentration of 100 micromolar from a sterile stock of 100 millimolar in distilled water. The broth was pre warmed to 25 °C and seeded with 1/10000th volume of an overnight tryptone soya broth culture, containing 2% salt, of *Photobacterium damselae* (strain MT1415, capsule positive virulent isolate as deposited under Accession No. 41062 on 4 August 2000 at N.C.I.M.B in Aberdeen, United Kingdom). Incubation was continued at 25 °C with shaking at 140 rpm for 40 hours. After incubation, the culture was inactivated by addition of 0.5% formalin v/v (0.2% free formaldehyde) and the broth was left at 25 °C for 24 hours to allow complete inactivation. The optical density of the final vaccine preparation was determined and the vaccine was stored at 4 °C until required.

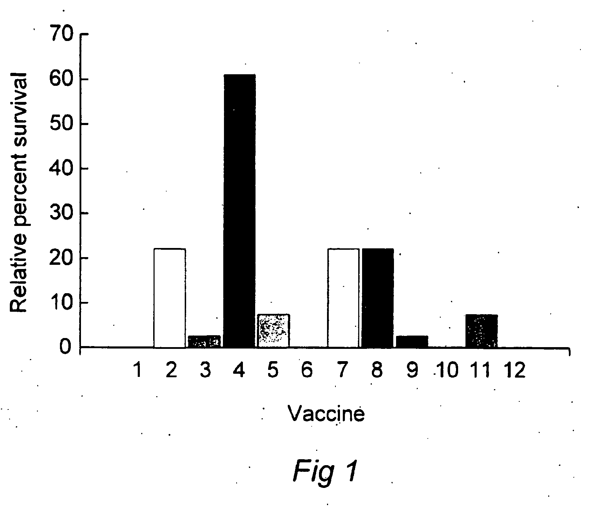

For administration this cell suspension was administered in one of a number of ways: Rainbow trout (10-15g) held in freshwater at 25 °C were anaesthetised with MS222 (sigma). Anaesthetised fish were vaccinated by intraperitoneal injection of neat vaccine preparation (100µl) or reference preparation or sterile phosphate buffered saline as a control. At least 500 degree days post immunisation, fish were challenged by injection of 10⁸ cfu of virulent *P. damselae* intraperitoneally. Mortalities were recorded daily and the RPS (relative percent survival) as compared to control fish was determined. Results are shown in Figure 1 below wherein the RPS using the present invention is compared with a number of reference vaccines. 1, reference vaccine; 2, reference vaccine; 3, Iron-restricted vaccine; 4, iron supplemented vaccine (vaccine of the invention); 5, standard tryptone soya broth (TSB) vaccine; 6-12, reference vaccines.

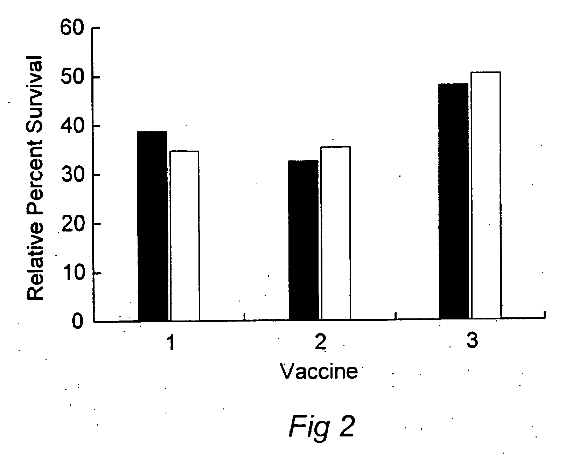

Alternatively, sea bream, 0.3 g were immunised by single 60 second immersion in a tenfold dilution of the present invention or reference vaccine. 500 degree days post vaccination fish were challenged by immersion in a suspension containing approximately 10⁵ cfu/ml virulent *P. damselae* for 1 hour at 25 °C. Mortalities were recorded as described above. Results are given in Figure 2: 1, iron restricted vaccine; 2, TSB vaccine; 3, Iron-supplemented vaccine (invention).

### Identification protective antigens

Vaccine prepared as described above was used to immunise sea bass (*Dicentrarchus labrax*) by intraperitoneal injection. Freunds incomplete adjuvant was administered simultaneously at a ratio of 1:1. Fish were maintained in seawater at 25 °C for 20 days after which they received a second identical dose. After a further 20 days fish were bled and sera collected by allowing the blood to clot and removing the red blood cells by centrifugation. Sera were dialysed against phosphate buffered saline and stored frozen at -80 °C.

Outer membrane proteins (OMP) and extracellular products (ECP) were prepared from *P. damselae* grown under iron limitation, in standard TSB, or under iron supplementation. For iron limitation, 2,2 dipyridyl (100 micromolar) was added to tryptone soya broth to chelate iron prior to inoculation with *P. damselae.* Incubation was then carried out as described above.OMP was prepared by precipitation following sarkosyl solubilisation of the inner membrane (Hancock & Poxton (1988) Bacterial Cell Surface Techniques, John Wiley & Sons, Chichester, UK). Extracellular products were recovered from broth culture supernatants.

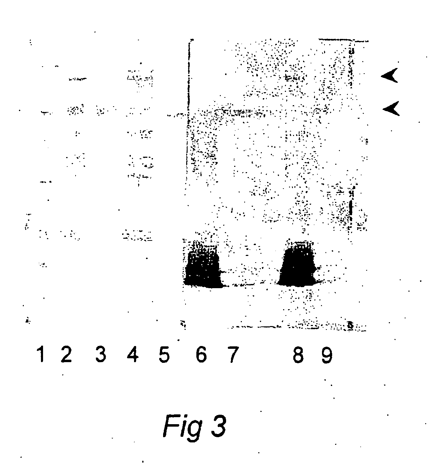

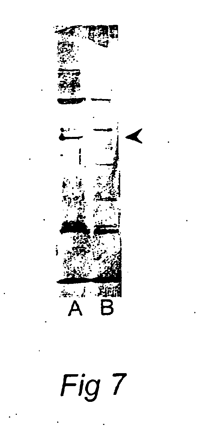

ECPs and OMP (equal protein concentrations) were run on SDS-PAGE gels under non-reducing conditions and blotted onto PVDF membrane. Membranes were probed with bass vs iron-supplemented *P. damselae*, followed by mouse vs bass immunoglobulin monoclonal antibody previously described (Santos et al. (1997) Fish. Shellfish Immunol.7:175-191), followed by goat vs mouse conjugated with an alkaline phosphatase enzyme. To visualise bands, membranes were incubated in substrate consisting of Nitroblue tetrazoleum and 5-Bromo-4-chloro-3-indolyl phosphate. The results are shown in figure 3 and figure 7. In the OMP preparations from iron containing cultures a clear band is visible close to the 97Kda marker which is not present under iron limitation (figures 3 and 7). By scanning densitometry this band was determined to be at least 2.6 fold more concentrated in OMPs from iron-supplemented cultures than from standard TSB cultures, and not detectable in iron limited cultures. In ECPs from cells grown in iron containing media, two bands were evident, one running close to 97Kda appeared to be the same as the band seen in OMP preparations. The other was smaller, running close to, but below, the 55KDa marker.Neither of these bands were detected in OMPS or ECP from cells cultured in iron deficient media.

### Inhibition of invasion of fish epithelial cells by antisera raised against iron-supplemented P. damselae

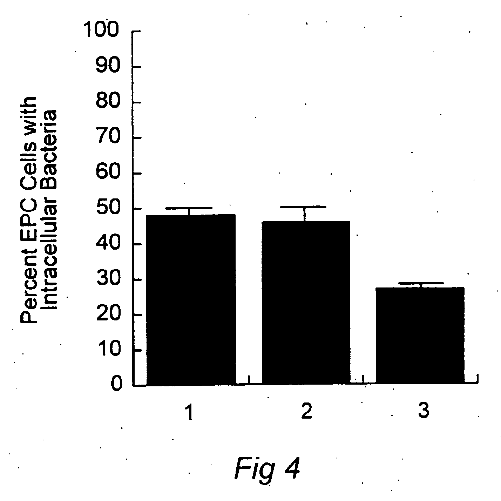

The ability of *P. damselae* to invade fish epithelial cells (EPC) was determined by a fluorescent labelling direct count method described by Bandin et al.(1995) Dis Aquat. Org. 23: 221-227. *P. damselae* cells were labelled using fluorescein isothiocyanate (FITC) (0.1mg/ml) for 1 hour. *P. damselae* were then washed extensively in PBS and resuspended to a density of 10⁹ cells /ml. The cell suspension was split and an aliquot incubated with heat inactivated immune serum prepared by heating sea bass vs iron-supplemented *P. damselae* antisera described above at 45 °C for 15 minutes, whilst a second aliquot was incubated with heat-inactivated normal sea bass serum. Aliquots (10 microlitres) of serum treated *P*.*damselae* cells were added to 6 x 10⁵ EPC cells in 1ml G-MEM, and allowed to attach and invade for 2 hours at 25 °C. External bacteria were removed by washing in PBS and EPCs were counter-stained with ethidium bromide. Aliquots (10 µl) were placed on glass slides and covered with a coverslip before analysis by fluorescent microscopy. Proportions of internalised bacteria were determined by direct counting at least 100 fields. Experiments were replicated four times. The results are presented in figure 4: 1, controls, *P. damaelae* with no serum; 2, *P. damselae* incubated with heat inactivated normal bass serum; 3, *P. damselae* incubated with heat inactivated antiserum (bass vs *P. damselae*)

### Identification of the protein associated with P. damselae internalisation in Sea Bass Cells using lectins

Previous work suggested that the entities involved in internalisation of *P. damselae* in fish cells may be glycoproteins (Magarios et al.(1996) FEMS Microbiol Lett. 138: 29-34). The carbohydrate side chains of glycoproteins can be specifically bound by certain lectins. Lectins are plant extracts with highly specific affinities for configurations of certain sugars. Incubating *P. damselae* with different lectins then determining its ability to invade sea bass larval fibroblast cells (SBL) identified lectins which were capable of inhibiting invasion and those which were not. The glycoproteins involved in invasion could then be identified by probing Western blots with biotinylated lectins.

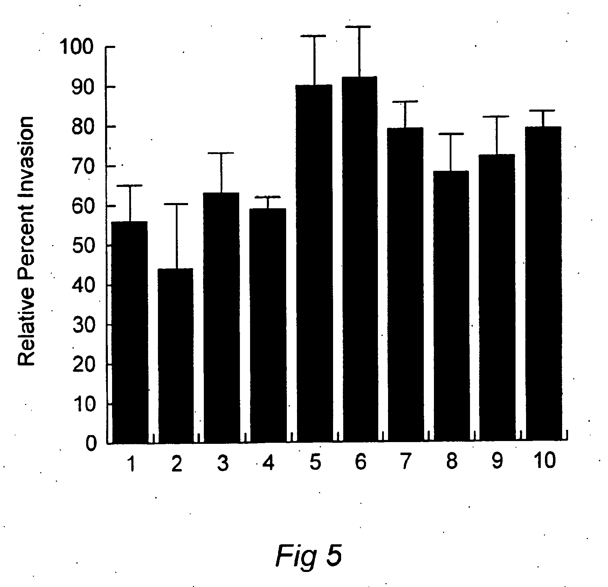

*P. damselae*, labelled with FITC, washed and resuspended to a density of 10⁹ cfu/ml as described above, was incubated for 1 hour with various lectins (Vector Laboratories) at a concentration of 100µg lectin/ml. After incubation, the cells were washed extensively and invasion assays carried out as described above. The results are presented in figure 5: *P.damselae* incubated with: 1, Sophora japonica agglutinin; 2, Concanavalin A agglutinin; 3, Lens culinaris agglutinin; 4, Griffonia simplicifolia agglutinin; 5, succinylated wheatgerm agglutinin; 6, Dolichos biflorus agglutinin; 7, peanut agglutinin; 8, soybean agglutinin; 9, Ulex europaeus agglutinin; 10, wheatgerm agglutinin.

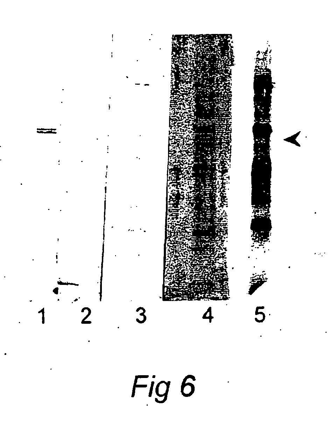

Two lectins which were able to strongly inhibit invasion by *P. damselae*, Sophora japonica agglutinin (SJA) and ConA agglutinin (ConA) were selected. One lectin which did not inhibit invasion, Dolichos biflorus agglutinin (DBA) was also selected. Biotinylated preparations of these lectins (Vector Laboratories) were used to stain Western blots of SDS-PAGE-separated OMPs from P. damselae. The results are presented in figure 6:
All the lectins stained a number of carbohydrates/glycoproteins. However, only one region was stained ConA, but not stained by DBA. This region consisted of a complex of three protein bands and had an approximate molecular weight of 55Kda under non reducing conditions and was only detected in OMP preps from *P. damselae* cultured under iron-replete conditions, not in preparations for *P. damselae* cultured under iron limitation. Furthermore, when Western blots of OMPs from *P. damselae* were cut and stained with both ConA lectin and sea bass vs iron-supplemented *P. damselae* antiserum a common band, the 55 Kda complex, was stained by both methods.Lane 1 shows OMPs probed with rabbit vs 55KDa complex, Lane 2 shows OMPs probed with bass vs Photobacterium damselae MT1415 antiserum used in invasion inhibition study. Lane 3 shows OMPS probed with dolichos biflorus agglutinin, Lane 4 shows OMPs probed with Concanavalin A lectin, Lane 5 shows Sigma biotinylated molecular weight markers (SDS-6B).

### Seralogical relationship between the 97 KDa OMP and the 55 KDa ECP.

The 97 KDa OMP and the 55KDa ECP were carefully excised from polyacrylamide gels, homogenised and injected into sea bass. After 30 days, sera were collected and used to probe Western blots of OMPs and ECPs. Antisera raised in sea bass against the 97Kda OMP cross reacted with the 55Kda ECP. Similarly, Antibodies raised in sea bass against the 55Kda ECP also cross reacted with the 97KDa OMP. The inventor suggests that the 55Kda ECP is a secreted version of the 97Kda OMP.

### Purification and N-terminal sequence

Subsequent purification of the 55Kda protein and sequencing have revealed three proteins in this region. The major antigenic protein is N-terminal blocked, consistant with glycosylation post transcription, and therefore unable to obtain a sequence, however this fraction has strong Haemagglutinating activity, suggesting probable involvement in internalisation. A second protein gave an N-terminal amino acid sequence with 100% homology to β-1,4 N-acetyl muramidase, a defence against other bacteria: AMKRHGLDNYRGYSLGNWVC.
The third protein may be a fragment of a deaminase or catabolic dehydratase : NVVLHGDNFDSTXVXVKAV.

### Comparison of protective efficacy of vaccines which express the 55KDa complex with vaccines which do not in a challenge study in Sea Bass (Dicentrarchus labrax).

The following study was performed independently at CEFAS Weymouth Laboratory under study protocol P0075, reference 99008).
Vaccines expressing the 55KDa protein complex were prepared as follows: 500 ml Tryptone soya broth +2% NaCl (TSB2) containing 200 micromolar ferric chloride in a 2.5 1 Erlenmeyer flask was inoculated with a 0.01% v/v inoculum of an 18h TSB2 culture of Photobacterium damseale subsp. piscicida MT1415. The culture was grown with shaking at 140 rpm until late exponential growth phase (about 40 hours) at 24 °C. The resulting culture was inactivated with formalin (final concentration 0.2%), and proteas.e was inactivated by adding Phenylmethylsulphonylfluoride (PMSF) to a final concentration of 100 micromolar from a 100 millimolar stock solution in isopropanol.

To prepare vaccines in which the expression of the 55KDa complex was completely inhibited. *Photobacterium damselae* subsp. *piscicida* isolate MT1415 was subcultureed twice for 18 hours in TSB2 containing 100 micromolar 2,2 dipyridyl, an iron chelator. This resulting completely iron-depleted culture was used as the inoculum (0.5%v/v) for the vaccine culture which was grown in 500 ml of TSB2 containing 100 micromolar 2,2 dipyridyl in a 2.5L. Erlenmeyer flask with shaking at 140 rpm until late exponential growth phase (about 48 hours) at 24 C. Absence of the 55Da complex from this preparation was confirmed by western blot of outer membrane proteins prepared from a duplicate culture (Refer to figure 7).

## Claims

1. A composition comprising biological material derived from a culture of Photobacterium damselae **characterised in that** the bacteria cells have been cultured in a culture medium containing at least twice the amount of iron as in standard tryptone soya broth, wherein said biological material includes: an outer membrane protein of approximately 97kDa as estimated by SDS-PAGE under non-reducing conditions which is involved in entry into host cells and/or an extracellular protein of approximately 55kDa as estimated by SDS-PAGE under non-reducing conditions and wherein the protein is expressed at levels at least twice the level of expression as in standard tryptone soy broth.

2. A composition according to claim 1 wherein outer membrane protein is invasin and/or the extracellular protein is serologically related to invasin.

3. A composition according to claim 1 wherein the outer membrane protein is adhesin.

4. A composition according to claim 1 wherein the outer membrane protein is a 97kDa outer membrane protein.

5. A composition according to claim 1 wherein the extracellular protein is a 55kDa extra cellular protein.

6. A composition according to claim 5 wherein said biological material comprising said outer membrane protein of approximately 97 kDa and said extracellular protein of approximately 55 kDa is produced by culture of Photobacterium clamselae MT1415 deposited under accession no 41062 at NCIMB in Aberdeen, UK and wherein the 55kDa extracellular protein is an extracellular protein complex comprising (i) an N-terminal blocked protein (ii) protein with sequence AMKRHGLDNYRGYSLGNWVC (iii) a protein with sequence of NVVLHGDNFDSTXVXVKAV.

7. A composition according to claim 1 wherein said composition comprises a 97kDa outer membrane protein and a 55kDa extracellular protein.

8. A composition according to claim 1 wherein said composition consists of a 97kDa outer membrane protein and a 55kDa extracellular protein.

9. A composition according to any one of claims 1-5, 7 and 8 wherein said composition comprises components produced by culture of Photobacterium damselae MT1415 deposited under accession no 41062 at NCIMB in Aberdeen, UK.

10. Use of a composition according to any one of claims 1-9 in the manufacture of a vaccine for the prophylactic and/or therapeutic treatment of fish infected by the organism Photobacterium damselae.

11. Use of the composition according to claim 10 in the manufacture of a vaccine for the prophylactic and/or therapeutic treatment of fish inflected by the organism Photobacterium damselae subspecies piscicida.

12. Use of a composition according to claim 10 or 11 for the prophylactic and or therapeutic treatment of pseudotuberculosis in fish:

13. Use according to any one of claims 10-12 wherein the fish are warm water marine fish.

14. Use according to claim 13 wherein the fish species are selected from rainbow trout, sea bream or sea bass.

15. Process for preparing a composition according to any one of claim 1-9 for use in a fish vaccine comprising: (i) culturing Photobacteria damselae cells in a culture medium containing at least twice the amount of iron as in standard tryptone soya broth and (ii) isolation of an outer membrane protein of Photobacteria damselae which is approximately 97kDa as estimated by SDS-PAGE under non-reducing conditions which is involved in entry into host cells and/or an extracellular protein of Photobacterium damselae which is approximately 55kDa as estimated by SDS-PAGE under non-reducing conditions.

16. Process according to claim 15 wherein in step (ii) the 97kDa outer membrane protein and/or, a 55kDa extracellular protein is isolated in the preparation of a fish vaccine.

17. Process according to claim 15 or claim 16 wherein an adjuvant suitable for enhancing immunological response is added.

18. Antibody raised against an outer membrane protein of Photobacteria damselae which is approximately 97kDa as estimated by SDS-PAGE under non-reducing conditions or raised against an extracellular, protein of Photobacterium damselae which is approximately 55kDa as estimated by SDS-PAGE under non-reducing conditions according to any one of claims 1-9.

19. Vaccine composition according to any one of claims 1-9 for the prophylactic and/or therapeutic use in fish infected by bacteria.

20. Vaccine composition according to claim 19 further comprising an adjuvant suitable for enhancing immunological response.

## Patentansprüche

1. Zusammensetzung, umfassen biologisches Material, abgeleitet von einer Kultur von Photobacteriujn damselae, **dadurch gekennzeichnet**, das die Bakterienzellen in einem Kulturmedium, enthaltend mindestens das Zweifache der Menge an Eisen, wie in einer Standard-Trypton-Sojabrühe, kultiviert wurden, wobei das biologische Material einschließt: ein äußeres Membranprotein von ungefähr 97 kDa, wie durch SDS-PAGE unter nicht reduzierenden Bedingungen geschätzt, das beim Eintritt in Wirtszellen einbezogen ist, und/oder ein extrazelluläres Protein von ungefähr 55 kDa, wie durch SDS-PAGE unter nicht reduzierenden Bedingungen geschätzt, und wobei das Protein mit Anteilen von mindestens dem Doppelten des Expressionsanteils, wie in Standard-Trypton-Sojabrühe, exprimiert wird.

2. Zusammensetzung nach Anspruch 1, worin das äußere Membranprotein Invasin und/oder das extrazelluläre Protein, das serologisch mit Invasin in Beziehung steht, darstellt.

3. Zusammensetzung nach Anspruch 1, worin das äußere Membranprotein Adhesin ist.

4. Zusammensetzung nach Anspruch 1, worin das äußere Membranprotein ein äußeres Membranprotein von 97 kDa ist.

5. Zusammensetzung nach Anspruch 1, worin das extrazelluläre Protein ein extrazelluläres Protein von 55 kDa ist.

6. Zusammensetzung nach Anspruch 5, worin das biologische Material, welches das äußere Membranprotein von ungefähr 97 kDa und das extrazelluläre Protein von ungefähr 55 kDa umfasst durch Kultur von *Photobacterium damselae MT1415,* hinterlegt unter der Zugangs-Nr. 41062 bei NCIMB in Aberdeen, GB, hergestellt wird und worin das extrazelluläre Protein von 55 kDa ein extrazellulärer Proteinkomplex ist, umfassend: (i) ein N-terminal blockiertes Protein, (ii) Protein mit Sequenz AMKRHGLDNYRGYSLGNWVC, (iii) Protein mit Sequenz NVVLHGDNFDSTXVXVKAV.

7. Zusammensetzung nach Anspruch 1, worin die Zusammensetzung ein äußeres Membranprotein von 97 kDa und ein extrazelluläres Protein von 55 kDa umfasst.

8. Zusammensetzung nach Anspruch 1, worin die Zusammensetzung aus einem äußeren Membranprotein von 97 kDa und einem extrazellulären Protein von 55 kDa besteht.

9. Zusammensetzung nach einem der Ansprüche 1-8, worin die Zusammensetzung Komponenten, erzeugt durch die Kultur von *Photobacterium damselae* MT1415, hinterlegt unter der Zugangs-Nr. 41062 bei NCIMB in Aberdeen, GB, hergestellt wird.

10. Verwendung einer Zusammensetzung nach einem der Ansprüche 1-9 bei der Herstellung eines Impfstoffs für die prophylaktische und/oder therapeutische Behandlung von Fisch, der durch den *Photobacterium damselae* infiziert wurde.

11. Verwendung einer Zusammensetzung nach Anspruch 10 bei der Herstellung eines Impfstoffs für die prophylaktische und/ oder therapeutische Behandlung von Fisch, der durch den Organismus *Photobacterium damselae*, Subspezies *piscicida*, infiziert wurde.

12. Verwendung einer Zusammensetzung nach Anspruch 10 oder 11 für die prophylaktische und/oder therapeutische Behandlung von Pseudotuberculose beim Fisch.

13. Verwendung nach einem der Ansprüche 10-12, wobei der Fisch ein Warmwassermeeresfisch ist.

14. Verwendung nach Anspruch 13, wobei die Fischspezies aus Regenbogenforelle, Seebrasse oder Seebarsch ausgewählt sind.

15. Verfahren zum Herstellen einer Zusammensetzung nach einem der Ansprüche 1-9 zur Verwendung in einem Fischimpfstoff, umfassend: (i) Kultivieren von *Photobacteria damselae-*Zellen in einem Kulturmedium, enthaltend mindestens das Zweifache der Menge an Eisen, wie in Standard-Trypton-Sojabrühe, und (ii) Isolierung von einem äußeren Membranprotein von *Photobacteria damselae*, das ungefähr 97 kDa, wie durch SDS-PAGE unter nicht reduzierenden Bedingungen geschätzt, ist, das beim Eintritt in Wirtszellen einbezogen ist, und/oder ein extrazelluläres Protein von *Photobacterium damselae*, das ungefähr 55 kDa, wie durch SDS-PAGE unter nicht reduzierenden Bedingungen geschätzt, ist.

16. Verfahren nach Anspruch 15, wobei in Schritt (ii) das außere Membranprotein von 97 kDa und/oder ein extrazelluläres Protein von 55 kDa bei der Herstellung eines Fischimpfstoffs isoliert wird.

17. Verfahren nach Anspruch 15 oder Anspruch 16, wobei ein Hilfsmittel, das zum Verstärken der immunologischen Realtion geeignet ist, zugesetzt wird.

18. Antikörper gegen ein äußeres Membranprotein, von *Photobacteria damselae*, das ungefähr 97 kDa, wie durch SDS-PAGE unter nicht reduzierenden Bedingungen geschätzt, ist, oder gegen ein extrazelluläres Protein von *Photobacterium damselae*, das ungefähr 55 kDa, wie durch SDS-PAGE unter nicht reduzierenden Bedingungen geschätzt, ist, gemäß einem der Ansprüche 1-9.

19. Impfstoffzusammensetzung nach einem der Ansprüche 1-9 für die prophylaktische und/oder therapeutische Verwendung bei Fisch, der durch Bakterien infiziert ist.

20. Impfstoffzusammensetzung nach Anspruch 19, weiterhin umfassend ein Hilfsmittel, das zum Verstärken einer immunologischen Reaktion geeignet ist.

## Revendications

1. Composition comprenant un matériau biologique dérivé d'une culture de *Photobacterium damselae* **caractérisée en ce que** les cellules bactériennes ont été mises en culture dans un milieu de culture contenant au moins deux fois la quantité de fer contenue dans un bouillon Tryptone Soja standard, **caractérisé en ce que** ledit matériau biologique comprend une protéine de membrane externe d'environ 97kDa tel qu'estimé par SDS-PAGE dans des conditions non-réductrices qui est impliquée dans l'entrée dans les cellules hôtes et/ou une protéine extracellulaire d'environ 55kDa tel qu'estimé par SDS-PAGE dans des conditions non-rédactrices et **caractérise en ce que** la protéine est exprimés selon des taux qui équivalent au moins à deux fois le taux d'expression constaté dans un bouillon Tryptone Soja standard.

2. Composition selon la revendication 1, **caractérisée en ce que** la protéine de membrane externe est l'invasine et/ou **en ce que** la protéine extracellulaire est sérologiquement apparentés à l'invasine.

3. Composition selon la revendication 1, **caractérisée en ce que** la protéine de membrane externe est l'adhésine.

4. Composition selon la revendications 1, **caractérisée en ce que** la protéine de membrane externe est une protéine de membrane externe de 97kDa.

5. Composition selon la revendication 1, **caractérisée en ce que** la protéine extracellulaire est une protéine extracellulaire de 55kDa.

6. Composition selon la revendication 5, **caractérisée en ce que** ledit matériau biologique comprenant ladite protéine de membrane externe d'environ 97 kna et ladite protéine extracellulaire d'environ 55 kDa est produite par la culture de *Photobacterium damselae* MT1415 déposé sous le numéro d'entrée 41062 auprès du NCIMB à Aberdeen, GB eten ce que la protéine extracellulaire de 55kDa est un complexe protéique extracellulaire comprenant: (i) une protéine bloquée au niveau du terminal N, (H) une protéine possédant une séquence AMKRHGLDNYRGYSLGNWVC (iii) une protéine possédant une séquence NVVLHGDNFDSTXVXVKAV.

7. Composition selon la revendication 1, **caractérisée en ce que** ladite composition comprend une protéine de membrane externe de 97kDa et une protéine extracellulaire de 55kDa.

8. Composition selon la revendication 1, **caractérisée en ce que** ladite composition consiste en une protéine de membrane externe de 97kDa et une protéine extracellulaire de 55kDa.

9. Composition selon l'une quelconque des revendications 1-5, 7 et, **caractérisée en ce que** ladite composition comprend des composants produits par culture de *Photobacterium damselae* MT1415 déposé sous le numéro d'accès 41062 auprès de NCIMB à Aberdeen, GB.

10. Utilisation d'une composition selon l'une quelconque des revendications 1 à 9 dans la fabrication d'un vaccin destiné au traitement prophylactique et/ou thérapeutique d'un poisson infecté par l'organisme *Photobacterium damselae.*

11. Utilisation d'une composition selon la revendication 10 dans la fabrication d'un vaccin destiné au traitement prophylactique et/ou thérapeutique d'un poisson infecté par la sous-espèce *piscicida* de l'organisme *Photobacterium damselae.*

12. Utilisation d'une composition selon la revendication 10 ou 11 pour le traitement prophylactique et/ou thérapeutique de la pseudotuberculose chez le poisson.

13. Utilisation d'une composition selon l'une quelconque des revendications 10 à 12, **caractérisée en ce que** le poisson est un poisson d'eaux chaudes.

14. Utilisation selon la revendications 13, **caractérisée en ce que** les espèces de poissons sont sélectionnées à partir de la truite arc-en-ciel, du spare tête-de-mouton ou du bar commun.

15. Procède de préparation d'une composition selon l'une quelconque des revendications 1 à 9 utilisable dans un vaccin destiné au poisson comprenant: (i) la mise en culture de cellules de *Photobacteria damselae* dans un milieu de culture contenant au moins deux fois la quantité de fer contenue dans un bouillon Tryptone Soja standard et (ii) l'isolation d'une protéine de membrane externe de *Photobacteria damselae* qui est d'environ 97kDa tel qu'estimé par SDS-PAGE dans des conditions non-réductrices qui est impliquée dans l'entrée dans les cellules hôtes et/ou d'une protéine extracellulaire de *Photobacterium damselae* qui est d'environ 55kDa tel qu'estime par SDS-PAGE dans des conditions non-réductrices.

16. Procédé selon la revendication 15, **caractérisé en ce que** lors de l'étape (ii) la protéine de membrane externe de 97kDa et/ou la protéine extracellulaire de 55kDa, est isolée lors de la préparation d'un vaccin destinée au traitement de poissons.

17. Procédé selon la revendication 15 ou 16, **caractérisé en ce qu'**un adjuvant convenant à l'accroissement de la réponse immunologique est ajouté.

18. Anticorps agissant à l'encontre d'une protéine de membrane externe de *Photobacteria damselae* qui est d'environ 97kDa tel qu'estimé par SDS-PAGE dans des conditions non-réductrices ou agissant à l'encontre d'une protéine extracellulaire de *Photobacterium damselae* qui est d'environ 55kDa tel qu'estimé par SDS-PAGE dans des conditions non-réductrices selon l'une quelconque des revendications 1 à 9.

19. Composition destinée à la préparation d'un vaccin selon l'une quelconque des revendications 1 à 9 destinée au traitement prophylactique et/ou thérapeutique d'un poisson infecté par des bactéries.

20. Composition destinée à la préparation d'un vaccin selon la revendication 19 comprenant également un adjuvant convenant à l'accroissement de la réponse immunologique.
